(19) Europäisches Patentamt
European Patent Office
Office européen des brevets

(11) Veröffentlichungsnummer: 0 351 649
A2

# (12) EUROPÄISCHE PATENTANMELDUNG

(21) Anmeldenummer: 89112306.9

(22) Anmeldetag: 06.07.89

(51) Int. Cl.4: C07D 249/08 , A01N 43/653 , C07D 303/08 , C07D 303/22 , C07D 303/34 , C07C 49/577 , C07C 49/567

(30) Priorität: 19.07.88 DE 3824435

(43) Veröffentlichungstag der Anmeldung: 24.01.90 Patentblatt 90/04

(84) Benannte Vertragsstaaten: BE CH DE ES FR GB IT LI NL

(71) Anmelder: BAYER AG

D-5090 Leverkusen 1 Bayerwerk(DE)

(72) Erfinder: Kraatz, Udo, Dr.
Andreasstrasse 22a
D-5090 Leverkusen 1(DE)
Erfinder: Holmwood, Graham, Dr.
Krutscheider Weg 105
D-5600 Wuppertal 11(DE)
Erfinder: Gassen, Karl-Rudolf, Dr.
Auenweg 6a
D-5068 Odenthal(DE)
Erfinder: Dutzmann, Stefan, Dr.
Leinenweberweg 33
D-4000 Düsseldorf 13(DE)
Erfinder: Brandes, Wilhelm, Dr.
Eichendorffstrasse 3
D-5653 Leichlingen(DE)

## (54) 2,2-Dihalogencyclopropyl-hydroxy-ethyltriazole.

(57) Neue 2,2-Dihalogencyclopropyl-hydroxy-ethyltriazole der Formel

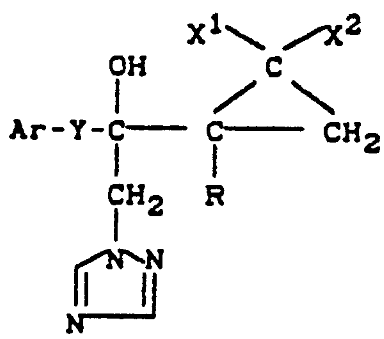

(I)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Halogen steht und
Y für die Gruppierungen $-OCH_2-$, $-SCH_2-$, $-CH_2CH-$ oder $-CH=CH-$ steht, wobei das Heteroatom mit dem Aryl-Rest verbunden ist, wenn Y für $-OCH_2-$ oder $-SCH_2-$steht,
sowie deren Säureadditions-Salze und Metallsalz-Komplexe,
ein Verfahren zur Herstellung der neuen Stoffe und deren Verwendung als Fungizide.

EP 0 351 649 A2

Neue Zwischenprodukte, Verfahren zu deren Herstellung und deren Verwendung zur Synthese von Stoffen der Formel (I).

## 2,2-Dihalogencyclopropyl-hydroxy-ethyltriazole

Die vorliegende Erfindung betrifft neue 2,2-Dihalogencyclopropyl-hydroxy-ethyltriazole, ein Verfahren zu ihrer Herstellung und deren Verwendung als Fungizide.

Es ist bereits bekannt, daß bestimmte Cyclopropyl-hydroxy-ethyltriazole fungizide Eigenschaften besitzen (vgl. EP-OS 0 040 345 und EP-OS 0 180 136). So lassen sich z.B. 1-(4-Chlorphenoxy)-2-cyclopropyl-3-(1,2,4-triazol-1-yl)-propan-2-ol, 1-(4-Chlorphenyl)-1-(1-chlorcycloprop-1-yl)-2-(1,2,4-triazol-1-yl)-ethan-1-ol und 1-(4-Chlorphenyl)-1-[1-(2,4-dichlorphenoxy)-cycloprop-1-yl]-ethan-1-ol zur Bekämpfung von Pilzen verwenden. Die Wirksamkeit dieser stoffe ist gut, läßt allerdings bei niedrigen Aufwandmengen in manchen Fällen zu wünschen übrig.

Es wurden nun neue 2,2-Dihalogencyclopropyl-hydroxy-ethyltriazole der Formel

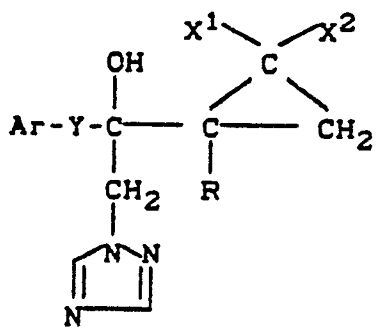

(I)

in welcher

Ar für gegebenenfalls substituiertes Aryl steht,

R für Alkyl, gegebenenfalls substituiertes Aryl oder gegebenenfalls substituiertes Aralkyl steht,

$X^1$ für Halogen steht,

$X^2$ für Halogen steht und

Y für die Gruppierungen $-OCH_2-$, $-SCH_2-$, $-CH_2CH_2-$ oder $-CH=CH-$ steht, wobei das Heteroatom mit dem Aryl-Rest verbunden ist, wenn Y für $-OCH_2-$ oder $-SCH_2-$steht,

sowie deren Säureadditions-Salze und Metallsalz-Komplexe gefunden.

Die erfindungsgemäßen Stoffe enthalten ein asymmetrisch substituiertes Kohlenstoffatom. Sie können daher in optischen Isomerenformen anfallen. Darüber hinaus können diejenigen Stoffe der Formel (I), in denen Y für eine -CH=CH-Gruppe steht, auch in Form von E- bzw. Z-Isomeren vorliegen. Die Erfindung betrifft sowohl die einzelnen Isomeren als auch deren Gemische.

Weiterhin wurde gefunden, daß man 2,2-Dihalogencyclopropyl-hydroxy-ethyltriazole der Formel (I) sowie deren Säureadditions-Salze und Metallsalz-Komplexe erhält, wenn man

Oxirane der Formel

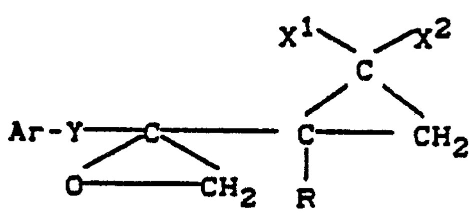

(II)

in welcher

Ar, R, $X^1$, $X^2$ und Y die oben angegebenen Bedeutungen haben,

mit 1,2,4-Triazol der Formel

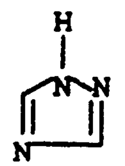

(III)

in Gegenwart eines Säurebindemittels und in Gegenwart eines Verdünnungsmittels umsetzt und gegebenenfalls anschließend an die so erhältlichen Verbindungen der Formel (I) eine Säure oder ein Metallsalz addiert.

Schließlich wurde gefunden, daß die neuen 2,2-Dihalogencyclopropyl-hydroxy-ethyltriazole der Formel (I) sowie deren Säureadditions-Salze und Metallsalz-Komplexe starke fungizide Eigenschaften besitzen.

Überraschenderweise zeigen die erfidungsgemäßen Stoffe eine bessere fungizide Wirksamkeit als 1-(4-Chlorphenoxy)-2-cyclopropyl-3-(1,2,4-triazol-1-yl)-propan-2-ol, 1-(4-Chlorphenyl)-1-(1-chlor-cycloprop-1-yl)-2-(1,2,4-triazol-1-yl)-ethan-1-ol und 1-(4-Chlorphenyl)-1-[1-2,4-dichlorphenoxy)-cycloprop-1-yl]-ethan-1-ol, welches konstitutionell ähnliche, vorbekannte Verbindungen gleicher Wirkungsrichtung sind.

Die erfindungsgemäßen 2,2-Dihalogencyclopropyl-hydroxy-ethyltriazole sind durch die Formel (I) allgemein definiert. In dieser Formel stehen vorzugsweise

Ar für Phenyl, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Alkoximinoalkyl mit 1 bis 4 Kohlenstoffatomen im Alkylteil und 1 bis 4 Kohlenstoffatomen im Alkoxyteil, gegebenenfalls durch Alkyl mit 1 oder 2 Kohlenstoffatomen und/oder Halogen substituiertes Phenoximinoalkyl mit 1 bis 4 Kohlenstoffatomen im Alkylteil, gegebenenfalls durch Alkyl mit 1 oder 2 Kohlenstoffatomen und/oder Halogen substituiertes Phenyl oder gegebenenfalls durch Alkyl mit 1 oder 2 Kohlenstoffatomen und/oder Halogen substituiertes Phenoxy,

R für Alkyl mit 1 bis 4 Kohlenstoffatomen, für Phenyl, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Alkoximinomethyl mit 1 bis 4 Kohlenstoffatomen im Alkoxyteil, und 1 bis 4 Kohlenstoffatomen im Alkoxyteil, gegebenenfalls durch Alkyl mit 1 oder 2 Kohlenstoffatomen und/oder Halogen substituiertes Phenoximinoalkyl mit 1 bis 4 Kohlenstoffatomen im Alkylteil, gegebenenfalls durch Alkyl mit 1 oder 2 Kohlenstoffatomen und/oder Halogen substituiertes Phenyl oder gegebenenfalls durch Alkyl mit 1 oder 2 Kohlenstoffatomen und/oder Halogen substituiertes Phenoxy, oder für Benzyl, das im Phenylteil einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Alkoximinoalkyl mit 1 bis 4 Kohlenstoffatomen im Alkylteil und 1 bis 4 Kohlenstoffatomen im Alkoxyteil, gegebenenfalls durch Alkyl mit 1 oder 2 Kohlenstoffatomen und/oder Halogen substituiertes Phenoximinoalkyl mit 1 bis 4 Kohlenstoffatomen im Alkylteil, gegebenenfalls durch Alkyl mit 1 oder 2 Kohlenstoffatomen und/oder Halogen substituiertes Phenyl oder gegebenenfalls durch Alkyl mit 1 oder 2 Kohlenstoffatomen und/oder Halogen substituiertes Phenoxy,

$X^1$ für Fluor, Chlor oder Brom,

$X^2$ für Fluor, Chlor oder Brom und

Y für die Gruppierungen $-OCH_2-$, $-SCH_2-$, $-CH_2CH_2-$ oder $-CH=CH-$, wobei das Heteroatom mit dem Aryl-Rest verbunden ist, wenn Y für $-OCH_2-$ oder $-SCH_2-$ steht.

Besonders bevorzugt sind diejenigen Verbindungen der Formel (I), in denen

Ar für Phenyl steht, das einfach bis dreifach, gleichartig oder verschieden substituiert sein kann durch Fluor, Chlor, Brom, Methyl, Ethyl, Isopropyl, tert.-Butyl, Methoxy, Methylthio, Trifluormethyl, Trifluormethoxy, Trifluormethylthio, Methoximinomethyl, 1-Methoximino-1-ethyl, Ethoximinomethyl, gegebenenfalls durch Fluor, Chlor und/oder Methyl substituiertes Phenyl oder gegebenenfalls durch Fluor, Chlor und/oder Methyl substituiertes Phenoxy;

R für Methyl, Ethyl, Isopropyl, tert.-Butyl steht, oder für gegebenenfalls einfach bis zweifach, gleich oder verschieden durch Fluor, Chlor oder Methyl substituiertes Phenyl steht, oder für Benzyl steht, das im Phenylteil einfach oder zweifach, gleichartig oder verschieden substituiert sein kann durch Fluor, Chlor und/oder Methyl,

$X^1$ für Fluor oder Chlor steht,

$X^2$ für Fluor oder Chlor steht und

Y für die Gruppierungen $-OCH_2-$, $-SCH_2-$, $-CH_2CH_2-$ oder $-CH=CH-$ steht, wobei das Heteroatom mit dem Aryl-Rest verbunden ist, wenn Y für $-OCH_2-$ oder $-SCH_2-$steht.

Ganz besonders bevorzugt sind diejenigen Verbindungen der Formel (I), in denen

Ar für Phenyl steht, das einfach bis dreifach, gleichartig oder verschieden substituiert sein kann durch Fluor, Chlor, Brom, Methyl, Ethyl, Isopropyl, tert.-Butyl, Methoxy, Methylthio, Trifluormethyl, Trifluormethoxy, Trifluormethylthio, Methoximinomethyl, 1-Methoximino-1-ethyl, Ethoximinomethyl, gegebenenfalls durch Fluor, Chlor und/oder Methyl substituiertes Phenyl oder gegebenenfalls durch Fluor, Chlor und/oder Methyl substituiertes Phenoxy,

R für Methyl, Ethyl, gegebenenfalls einfach oder zweifach durch Fluor, Chlor und/oder Methyl substituiertes Phenyl oder für Benzyl steht,

$X^1$ für Fluor oder Chlor steht,

$X^2$ für Fluor oder Chlor steht und

Y für die Gruppierung $-OCH_2-$, $-SCH_2-$, $-CH_2CH_2-$ oder $-CH=CH-$ steht, wobie das Heteroatom mit dem Aryl- Rest verbunden ist, wenn Y für $-OCH_2-$ oder $-SCH_2-$steht.

Bevorzugte erfindungsgemäße Verbindungen sind auch Additionsprodukte aus Säuren und denjenigen 2,2-Dihalogencyclopropyl-hydroxy-ethyltriazolen der Formel (I), in denen Ar, R, $X^1$, $X^2$ und Y die Bedeutungen haben, die bereits vorzugsweise für diese Reste genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen 2,2-Dihalogencyclopropyl-hydroxy-ethyltriazolen der Formel (I), in denen Ar, R, $X^1$, $X^2$ und Y die Bedeutungen haben, die bereits vorzugsweise für diese Reste genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen.

Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für 2,2-Dihalogencyclopropyl-hydroxy-ethyltriazole der Formel (I) seien die in der folgenden Tabelle aufgeführten Stoffe genannt:

Tabelle 1

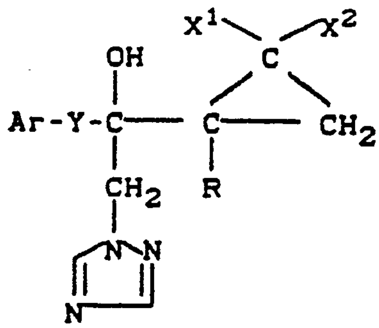

(I)

| Ar | Y | R | $X^1$ | $X^2$ |
|---|---|---|---|---|
| 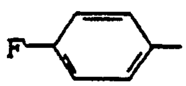 | $-OCH_2-$ | $CH_3$ | F | F |
| 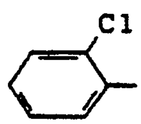 | $-OCH_2-$ | $CH_3$ | F | F |
| 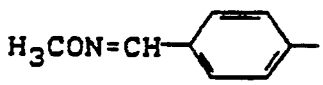 | $-OCH_2-$ | $CH_3$ | F | F |
| 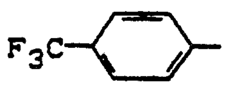 | $-OCH_2-$ | $CH_3$ | F | F |
| 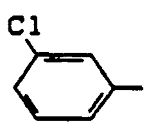 | $-OCH_2-$ | $CH_3$ | F | F |
| 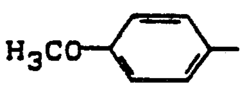 | $-OCH_2-$ | $CH_3$ | F | F |
| 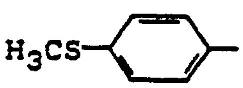 | $-OCH_2-$ | $CH_3$ | F | F |

Tabelle 1 (Fortsetzung)

| Ar | Y | R | $X^1$ | $X^2$ |
|---|---|---|---|---|
| 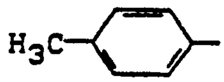 | $-OCH_2-$ | $CH_3$ | F | F |
| 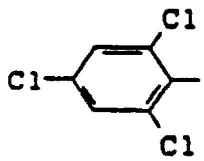 | $-OCH_2-$ | $CH_3$ | F | F |
| 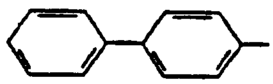 | $-OCH_2-$ | $CH_3$ | F | F |
| 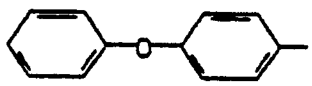 | $-OCH_2-$ | $CH_3$ | F | F |
| 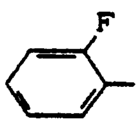 | $-OCH_2-$ | $CH_3$ | F | F |
| 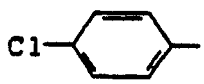 | $-SCH_2-$ | $CH_3$ | F | F |
| 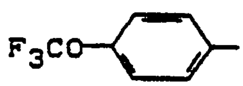 | $-SCH_2-$ | $CH_3$ | F | F |
| 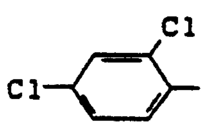 | $-SCH_2-$ | $CH_3$ | F | F |
| 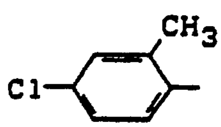 | $-SCH_2-$ | $CH_3$ | F | F |
| 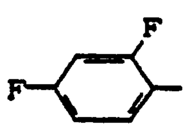 | $-SCH_2-$ | $CH_3$ | F | F |

Tabelle 1 (Fortsetzung)

| Ar | Y | R | $X^1$ | $X^2$ |
|---|---|---|---|---|
| 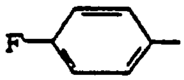 | $-SCH_2-$ | $CH_3$ | F | F |
| 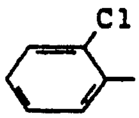 | $-SCH_2-$ | $CH_3$ | F | F |
| 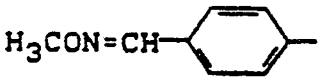 | $-SCH_2-$ | $CH_3$ | F | F |
| 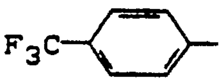 | $-SCH_2-$ | $CH_3$ | F | F |
| 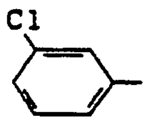 | $-SCH_2-$ | $CH_3$ | F | F |
| 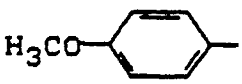 | $-SCH_2-$ | $CH_3$ | F | F |
| 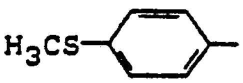 | $-SCH_2-$ | $CH_3$ | F | F |
| 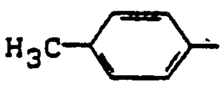 | $-SCH_2-$ | $CH_3$ | F | F |
| 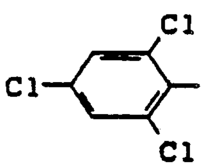 | $-SCH_2-$ | $CH_3$ | F | F |
| 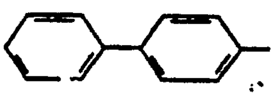 | $-SCH_2-$ | $CH_3$ | F | F |
| 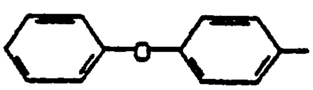 | $-SCH_2-$ | $CH_3$ | F | F |
| 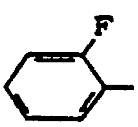 | $-SCH_2-$ | $CH_3$ | F | F |

Tabelle 1 (Fortsetzung)

| Ar | Y | R | $X^1$ | $X^2$ |
|---|---|---|---|---|
| 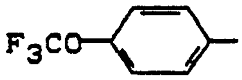 | $-CH_2CH_2-$ | $CH_3$ | F | F |
| 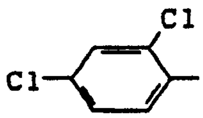 | $-CH_2CH_2-$ | $CH_3$ | F | F |
| 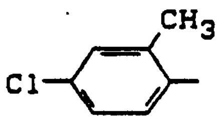 | $-CH_2CH_2-$ | $CH_3$ | F | F |
| 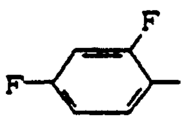 | $-CH_2CH_2-$ | $CH_3$ | F | F |
| 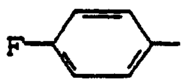 | $-CH_2CH_2-$ | $CH_3$ | F | F |
| 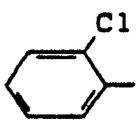 | $-CH_2CH_2-$ | $CH_3$ | F | F |
| 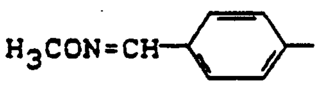 | $-CH_2CH_2-$ | $CH_3$ | F | F |
| 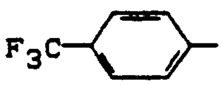 | $-CH_2CH_2-$ | $CH_3$ | F | F |
| 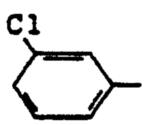 | $-CH_2CH_2-$ | $CH_3$ | F | F |
| 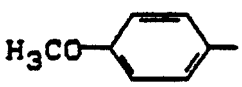 | $-CH_2CH_2-$ | $CH_3$ | F | F |
| 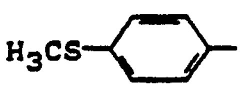 | $-CH_2CH_2-$ | $CH_3$ | F | F |
| 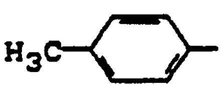 | $-CH_2CH_2-$ | $CH_3$ | F | F |

<u>Tabelle 1</u> (Fortsetzung)

| Ar | Y | R | $X^1$ | $X^2$ |
|---|---|---|---|---|
| 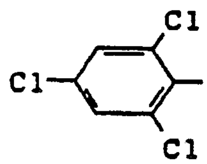 | $-CH_2CH_2-$ | $CH_3$ | F | F |
| 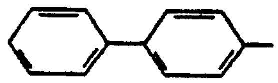 | $-CH_2CH_2-$ | $CH_3$ | F | F |
| 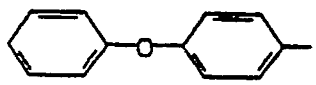 | $-CH_2CH_2-$ | $CH_3$ | F | F |
| 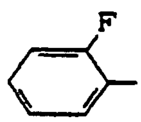 | $-CH_2CH_2-$ | $CH_3$ | F | F |
| 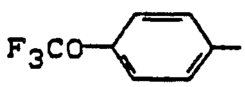 | -CH=CH- | $CH_3$ | F | F |
| 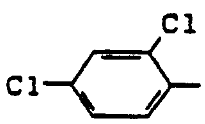 | -CH=CH- | $CH_3$ | F | F |
| 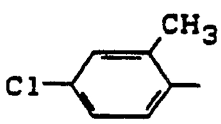 | -CH=CH- | $CH_3$ | F | F |
| 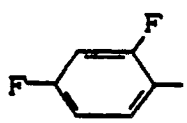 | -CH=CH- | $CH_3$ | F | F |
| 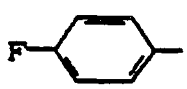 | -CH=CH- | $CH_3$ | F | F |
| 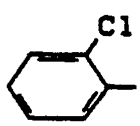 | -CH=CH- | $CH_3$ | F | F |
| 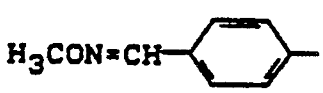 | -CH=CH- | $CH_3$ | F | F |

Tabelle 1 (Fortsetzung)

| Ar | Y | R | $X^1$ | $X^2$ |
|---|---|---|---|---|
| 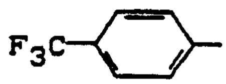 | -CH=CH- | $CH_3$ | F | F |
| 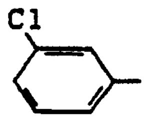 | -CH=CH- | $CH_3$ | F | F |
| 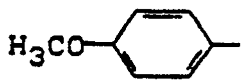 | -CH=CH- | $CH_3$ | F | F |
| 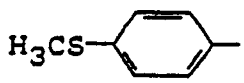 | -CH=CH- | $CH_3$ | F | F |
| 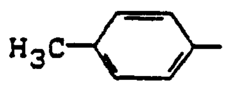 | -CH=CH- | $CH_3$ | F | F |
| 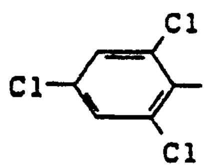 | -CH=CH- | $CH_3$ | F | F |
| 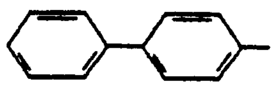 | -CH=CH- | $CH_3$ | F | F |
| 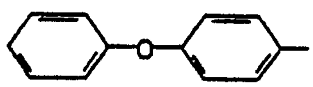 | -CH=CH- | $CH_3$ | F | F |
| 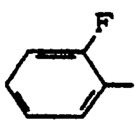 | -CH=CH- | $CH_3$ | F | F |

Verwendet man 2-(4-Chlorphenoxymethyl)-2-(2,2-difluor-1-methylcyclopropyl)-oxiran und 1,2,4-Triazol als Ausgangsstoffe, so kann der Verlauf des erfindungsgemäßen Verfahrens durch das folgende Formelschema veranschaulicht werden:

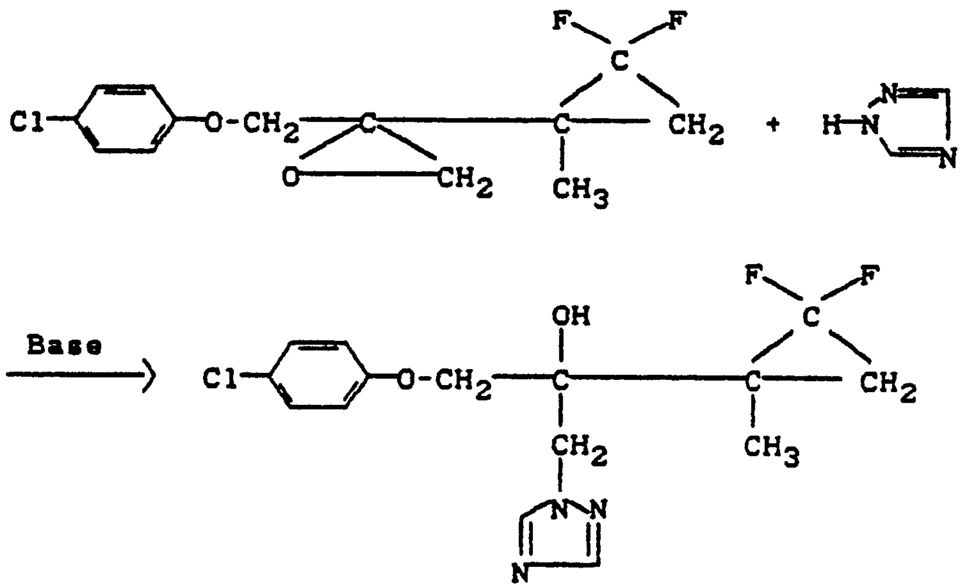


Die bei dem erfindungsgemäßen Verfahren als Ausgangsstoffe benötigten Oxirane sind durch die Formel (II) allgemein definiert. In dieser Formel haben Ar, R, $X^1$, $X^2$ und Y vorzugsweise diejenigen Bedeutungen, die bereits im Zusammenhang mit der Beschreibung der erfindungsgemäßen Stoffe der Formel (I) vorzugsweise für diese Reste genannt wurden.

Die Oxirane der Formel (II) sind bisher noch nicht bekannt. Sie lassen sich herstellen, indem man

a) Cyclopropylketone der Formel

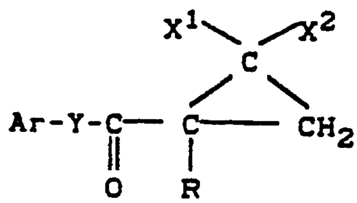 (IV)

in welcher

Ar, R, $X^1$, $X^2$ und Y die oben angegebene Bedeutung haben,

entweder

α) mit Dimethyloxosulfonium-methylid der Formel

$$\overset{\delta^{\oplus}\delta^{\ominus}}{(CH_3)_2SOCH_2} \qquad (V)$$

oder

β) mit Dimethylsulfonium-methylid der Formel

$$\overset{\delta^{\oplus}\delta^{\ominus}}{(CH_3)_2S\ CH_2} \qquad (VI)$$

in Gegenwart eines Verdünnungsmittels umsetzt.

Die bei der Durchführung des Verfahrens (a) als Ausgangsstoffe benötigten Cyclopropylketone der Formel (IV) sind bisher noch nicht bekannt. Sie lassen sich herstellen, indem man

b) Halogenketone der Formel

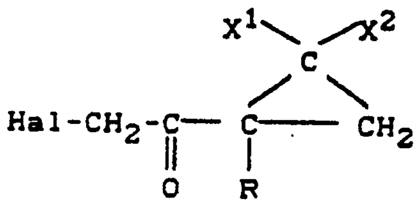

(VII)

in welcher
Hal für Chlor oder Brom steht und
R, $X^1$ und $X^2$ die oben angegebenen Bedeutungen haben,
mit Verbindungen der Formel
Ar - Z - H (VIII)
in welcher
Ar die oben angegebene Bedeutung hat und
Z für Sauerstoff oder Schwefel steht,
in Gegenwart eines Säurebindemittels und gegebenenfalls in Gegenwart eines Verdünnungsmittels umsetzt; oder
c) Aldehyde der Formel
Ar - CHO (IX)
in welcher
Ar die oben angegebene Bedeutung hat,
mit Methyl-cyclopropyl-ketonen der Formel

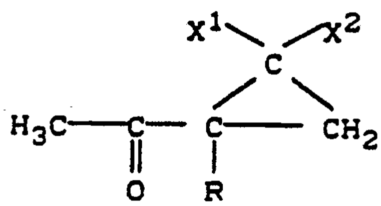

(X)

in welcher
R, $X^1$ und $X^2$ die oben angegebenen Bedeutungen haben,
in Gegenwart eines Katalysators und in Gegenwart eines Verdünnungsmittels umsetzt und gegebenenfalls die dabei entstehenden Cyclopropylketone der Formel

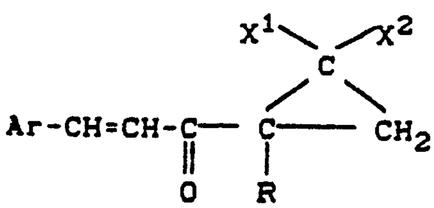

(XI)

in welcher
Ar, R, $X^1$ und $X^2$ die oben angegebenen Bedeutungen haben,
in Gegenwart eines Katalysators und in Gegenwart eines Verdünnungsmittels hydriert.

Die bei dem Verfahren (b) als Ausgangsstoffe benötigten Halogenketone der Formel (VII) sind noch nicht bekannt. Sie lassen sich herstellen, indem man
d) Methyl-cyclopropyl-ketone der Formel

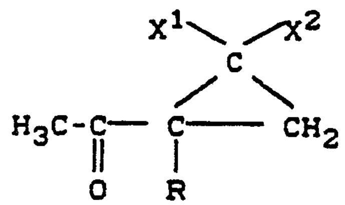

(X)

in welcher
R, $X^1$ und $X^2$ die oben angegebenen Bedeutungen haben,
mit Chlorierungsmitteln oder Bromierungsmitteln in Gegenwart eines Verdünnungsmittels umsetzt.

Die beim den Verfahren (c) und (d) als Ausgangsstoffe benötigten Ketone der Formel (X) sind ebenfalls noch nicht bekannt. Sie lassen sich herstellen, indem man Vinylcyclopropan-Derivate der Formel

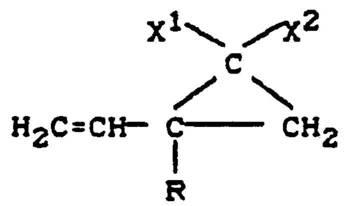

(XII)

in welcher
R, $X^1$ und $X^2$ die oben angegebenen Bedeutungen haben,
mit starken Oxidationsmitteln, wie z.B. Kaliumpermanganat, in Gegenwart eines Verdünnungsmittels, wie z.B. Wasser, bei Temperaturen zwischen 0 °C und 30 °C umsetzt und die entstehenden Cyclopropancarbonsäuren der Formel

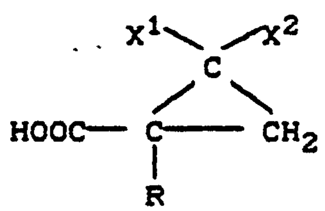

(XIII)

in welcher
R, $X^1$ und $X^2$ die oben angegebenen Bedeutungen haben,
mit Methyl-lithium in Gegenwart eines Verdünnungsmittels, wie z.B. Diethylether, bei Temperaturen zwischen -80 °C und +20 °C umsetzt.

Die Vinylcyclopropan-Derivate der Formel (XII) sind teilweise bekannt (vgl. Chem. Ber. 109, 2351 (1976)); bzw. lassen sie sich in allgemein bekannter Art und Weise herstellen, z.B. durch Addition von Dihalogencarbenen an entsprechende Diene.

Als Chlorierungsmittel und Bromierungsmittel kommen bei dem Verfahren (d) alle für derartige Umsetzungen üblichen Chlorierungs- und Bromierungsreagenzien in Betracht. Vorzugsweise verwendbar sind Sulfurylchlorid, Sulfurylbromid und Brom.

Als Verdünnungsmittel kommen bei dem Verfahren (d) alle für derartige Umsetzungen üblichen inerten organischen Solventien in Frage. Vorzugsweise verwendbar sind halogenierte aliphatische Kohlenwasserstoffe, wie Methylenchlorid, Chloroform und Tetrachlorkohlenstoff.

Die Reaktionstemperaturen können bei dem Verfahren (d) innerhalb eines bestimmten Bereiches variiert werden. Im allgemeinen arbeitet man bei Temperaturen zwischen -10 °C und +60 °C, vorzugsweise zwischen 0 °C und +40 °C.

Bei der Durchführung des Verfahrens (d) arbeitet man ebenso wie bei den übrigen in dieser Anmeldung beschrieben Verfahren im allgemeinen unter Normaldruck. Es ist jedoch jeweils auch möglich, unter erhöhtem oder vermindertem Druck zu arbeiten.

Bei der Durchführung des Verfahrens (d) setzt man auf 1 Mol an Keton der Formel (X) im allgemeinen eine stöchiometrische Menge oder auch einen geringen Überschuß an Chlorierungs- oder Bromierungsmittel ein. Die Aufarbeitung erfolgt nach üblichen Methoden. Im allgemeinen geht man so vor, daß man das

Reaktionsgemisch nacheinander mit verdünnter, wäßriger Natriumhydrogencarbonat-Lösung und Wasser wäscht, dann trocknet und einengt.

Die außerdem bei dem Verfahren (c) als Ausgangsstoffe benötigten Aldehyde der Formel (IX) sind allgemein bekannte Verbindungen der organischen Chemie.

Als Katalysatoren kommen bei der Durchführung der ersten Stufe des Verfahrens (c) alle für derartige Kondensationen üblichen Reaktionsbeschleuniger in Frage. Vorzugsweise verwendbar sind basische Substanzen, z.B. Alkalimetallhydroxide, wie Natriumhydroxid oder Kaliumhydroxid.

Als Verdünnungsmittel kommen bei der Durchführung der ersten Stufe des Verfahrens (c) alle für derartige Umsetzungen üblichen inerten organischen Solventien in Betracht. Vorzugsweise verwendbar sind Alkohole, wie Methanol, Ethanol, Isopropanol, n-Butanol und tert.-Butanol.

Die Reaktionstemperaturen können bei der Durchführung der ersten Stufe des Verfahrens (c) innerhalb eines größeren Bereiches variiert werden. Im allgemeinen arbeitet man bei Temperaturen zwischen 0°C und 100°C, vorzugsweise zwischen 10°C und 80°C.

Die erste Stufe des Verfahrens (c) wird im allgemeinen unter Normaldruck durchgeführt. Es ist aber auch möglich, unter erhöhtem oder vermindertem Druck zu arbeiten.

Bei der Durchführung der ersten Stufe des Verfahrens (c) setzt man auf 1 Mol an Methyl-cyclopropylketon der Formel (X) 1 Mol an Aldehyd der Formel (IX) sowie eine katalytische Menge an Reaktionsbeschleuniger ein. Es ist jedoch auch möglich, die eine oder andere Komponente in einem Überschuß zu verwenden. Die Aufarbeitung erfolgt nach üblichen Methoden. Im allgemeinen geht man so vor, daß man die in festem Zustand anfallenden Reaktionsprodukte absaugt und nach gegebenenfalls vorheriger Reinigung für die weiteren Umsetzungen verwendet.

In der zweiten Stufe des Verfahrens (c) werden die Cyclopropylketone der Formel (XI) Gegenwart eines Katalysators und eines Verdünnungsmittels mit Wasserstoff hydriert. Man arbeitet dabei in flüssiger Phase unter Verwendung eines suspendierten, pulverförmigen Hydrierkatalysators (heterogen) oder unter Verwendung eines im Verdünnungsmittel löslichen Katalysatorkomplexes (homogen). Die Durchführung der Hydrierung kann diskontinuierlich (chargenweise) oder kontinuierlich als Sumpf- oder Rieselphasenhydrierung in bekannten Hydrierreaktoren, wie Autoklaven, Autoklavenkaskaden, Rohrreaktoren oder Umlaufreaktoren erfolgen. Die bevorzugte Arbeitsweise ist die diskontinuierliche Sumpfphasenhydrierung im Autoklaven bei erhöhtem Druck.

Als Verdünnungsmittel kommen bei der Durchführung der zweiten Stufe des Verfahrens (c) inerte organische Lösungsmittel in Frage. Hierzu gehören vorzugsweise Alkohole, wie Methanol, Ethanol, Isopropanol oder Ethylenglykol; Ether, wie Diethylether, Diisopropylether, Ethylenglykolmonomethylether, Ethylenglykoldimethylether, Dioxan oder Tetrahydrofuran; gesättigte Kohlenwasserstoffe, wie n-Heptan oder Cyclohexan; aromatische Kohlenwasserstoffe, wie Toluol; sowie Ester, wie Essigsäureethylester.

Für die zweite Stufe des Verfahrens (c) geeignete Hydrierkatalysatoren sind beispielsweise solche, die aus Metallen und/oder Verbindungen von Elementen der achten Nebengruppe des periodischen Systems der Elemente nach Mendelejew bestehen oder diese enthalten. Dabei sind die Metalle Ruthenium, Rhodium, Palladium, Platin, Kobalt und Nickel und deren Verbindungen bevorzugt. Bei den Metallverbindungen kann es sich beispielsweise um Chloride, Oxide, Hydroxide und/oder Oxihydrate handeln.

Zusätzlich können die Metalle Kupfer, Vanadium, Molybdän, Chrom und/oder Mangan, sowie Verbindungen dieser Metalle zugegen sein.

Die Hydrierkatalysatoren können ausschließlich oder überwiegend aus Wasserstoff-übertragenden Substanzen bestehen, diese können aber auch auf Trägermaterialien aufgebracht sein.

Als Trägermaterialien für die Wasserstoff-übertragenden Substanzen kommen beispielsweise in Frage: anorganische Materialien, wie Kieselgur, Kieselsäure, Aluminiumoxid, Alkali- und Erdalkalisilikate, Aluminiumsilikate, Montmorillonit, Zeolithe, Spinelle, Dolomit, Kaolin, Magnesiumsilikate, Zirkonoxid, Zinkoxid, Calciumcarbonat, Siliciumcarbid, Aluminiumphosphat, Borphosphat, Asbest, Aktivkohle oder Bariumsulfat, aber auch organische Materialien, beispielsweise natürlich vorkommende oder synthetische Verbindungen mit hohen Molekulargewichten wie Seide, Polyamide, Polystyrole, Zellstoff oder Polyurethane. Bevorzugt sind anorganische Trägermaterialien in Pulverform.

Derartige Trägerkatalysatoren können im allgemeinen 0,5 bis 50 Gew-%, vorzugsweise 1 bis 10 Gew.-% der Wasserstoff-übertragenden Substanz, bezogen auf die Gesamtmasse des Trägerkatalysators, enthalten. Die Wasserstoff-übertagende Substanz kann dabei homogen im Trägermaterial verteilt sein, bevorzugt sind jedoch Katalysatoren, in deren äußerer Schicht oder auf deren Oberfläche die Wasserstoff-übertragende Substanz abgelagert ist. Die Herstellung und die Formgebung der Katalysatoren, die im Verfahren (c) Verwendung finden können, kann in bekannter Weise erfolgen (siehe beispielsweise Houben-Weyl, Methoden der organischen Chemie, Band IV, Ic, Teil I, S. 16 bis 26, Georg Thieme Verlag, Stuttgart, 1980).

Bevorzugte Trägerkatalysatoren sind Ruthenium auf Kohle, Ruthenium auf Aluminiumoxid, Rhodium auf

Kohle, Rhodium auf Aluminiumoxid, Palladium auf Kohle, Palladium auf Aluminiumoxid, Palladium auf Calciumcarbonat, Palladium auf Bariumsulfat, Palladium auf Kieselsäure, Platin auf Kohle und Platin auf Aluminiumoxid, Nickel auf Kieselgur, Nickel auf Aluminiumoxid sowie Nickel und Palladium auf Aluminiumoxid.

Bei Hydrierung im heterogenen System bevorzugte Hydrierkatalysatoren, die ausschließlich oder überwiegend aus Wasserstoff-übertragender Substanz bestehen, sind beispielsweise oxidische Katalysatoren, wie Palladiumoxid, Platinoxid, Rutheniumoxid und/oder Rhodiumoxid/Platinoxid nach Nishimura, ferner durch Reduktion von entsprechenden Metallsalzen oder Metallsalzgemischen mit Alkalihydriden, Alkaliboranaten, Metallalkylen, Hydrazin, Formaldehyd, Wasserstoff oder elektropositiven Metallen herstellbare Schwarzkatalysatoren, wie Palladium/Schwarz, Platin/Schwarz und Rhodium/Schwarz; sowie Skelettkatalysatoren vom Raney-Typ, wie Raney-Nickel, Raney-Kobalt, Raney-Nickel-Kobalt, Raney-Nickel-Eisen, Raney-Nickel-Kupfer, Raney-Nickel-Eisen-Chrom, Raney-Nickel-Palladium und Raney-Nickel-Eisen-Vanadium.

Bei Hydrierung im heterogenen System werden die Hydrierkatalysatoren bei der zweiten Stufe des Verfahrens (c) in einer solchen Menge eingesetzt, daß 0,05 bis 2,5, vorzugsweise 0,1 bis 1 Gew.-% Wasserstoff-übertragende Substanz bezogen auf das Gesamtgewicht des Reaktionsgemisches vorliegen.

Zur Durchführung der zweiten Stufe des Verfahrens (c) können auch Gemische aus zwei oder mehreren der genannten Hydrierkatalysatoren verwendet werden.

Die katalytische Aktivität der Hydrierkatalysatoren bleibt bei der Durchführung der zweiten Stufe des Verfahrens (c) im allgemeinen weitgehend erhalten, so daß diese bei diskontinuierlicher Arbeitsweise wiederholt eingesetzt werden können und bei kontinuierlicher Arbeitsweise längere Zeit in Gebrauch bleiben können.

Die Reaktionstemperaturen können in der zweiten Stufe des Verfahrens (c) in einem größeren Bereich variiert werden. Im allgemeinen arbeitet man im Bereich zwischen 0 °C und 150 °C, vorzugsweise zwischen 20 °C und 120 °C.

Die heterogen katalysierten Hydrierungen in der zweiten Stufe des Verfahren (c) werden vorzugsweise bei erhöhtem Druck durchgeführt. Im allgemeinen arbeitet man zwischen 1 und 150 bar, vorzugsweise zwischen 10 und 60 bar.

Außer den genannten Hydrierkatalysatoren heterogener Natur können auch homogen gelöste Hydrierkatalysatoren bei der Durchführung der zweiten Stufe des Verfahrens (c) eingesetzt werden. Die im Vergleich zu heterogenen Katalysatoren oft höhere Selektivität homogener Hydrierkatalysatoren erlaubt die selektive Hydrierung von Cyclopropylketonen der Formel (XI), die zusätzliche hydrierbare oder hydrolyseempfindliche Substituenten, wie z.B. Halogen am Phenylrest, enthalten. Solche homogenen Hydrierkatalysatoren sind beispielsweise Komplexe, die Metalle der achten Nebengruppe des Periodensystems der Elemente nach Mendelejew als Zentralatom enthalten. Bevorzugt sind dabei die Metalle Ruthenium, Rhodium, Palladium, Iridium, Kobalt und Nickel. Besonders bevorzugt sind Ruthenium, Rhodium und Iridium. Als Beispiele für derartige Metallkomplexe seien Tris-(triphenylphosphin)-rhodium(I)-chlorid, Tris-(triphenylphosphin)-ruthenium(II)-chlorid und Bis-(triphenylphosphin)-carbonyl-iridium(I)-chlorid genannt.

Bei Verwendung homogen gelöster Hydrierkatalysatoren kommen bei der Durchführung der zweiten Stufe des Verfahrens (c) als Verdünnungsmittel inerte organische Solventien in Frage. Vorzugsweise verwendbar sind Alkohole, wie Methanol, Ethanol, Isopropanol oder Ethylenglykol, ferner Kohlenwasserstoffe, wie Toluol, außerdem Ketone, wie Aceton und Butanon, und auch Ester, wie Essigsäureethylester.

Bei der Hydrierung im homogenen System werden die Hydrierkatalysatoren bei der Durchführung der zweiten Stufe des Verfahrens (c) im allgemeinen in einer solchen Menge eingesetzt, daß 0,01 bis 2,5 Mol-%, vorzugsweise 0,05 bis 1,0 Mol-% an Hydrierkatalysator-Komplex bezogen auf eingesetztes Cyclopropylketon der Formel (XI) vorhanden sind.

Die Reaktionstemperaturen können auch bei der Hydrierung im homogenen System bei der Durchführung der zweiten Stufe des Verfahrens (c) innerhalb eines größeren Be reiches variiert werden. Im allgemeinen arbeitet man bei Temperaturen zwischen 0 °C und 150 °C, vorzugsweise zwischen 20 °C und 120 °C.

Die im homogenen System durchgeführten Hydrierungen in der zweiten Stufe des Verfahrens (c) werden vorzugsweise unter erhöhtem Druck vorgenommen. Im allgemeinen arbeitet man unter Drucken zwischen 1 und 150 bar, vorzugsweise zwischen 10 und 100 bar.

In einer Variante kann die Hydrierung im homogenen System in der zweiten Stufe des Verfahrens (c) auch so durchgeführt werden, daß nicht mit molekularem Wasserstoff hydriert wird, sondern daß Reduktionsmittel eingesetzt werden, die in der Lage sind, in Gegenwart eines geeigneten Katalysators ein oder mehrere Wasserstoffatome an das Cyclopropylketon der Formel (XI) im Sinne einer Transferhydrierung zu übertragen und daher als Wasserstoffdonatoren wirken. Als Katalysatoren für eine solche Transferhydrierung kommen prinzipiell die bereits für die homogen katalysierte Hydrierung mit molekularem Wasserstoff

in der zweiten Stufe des Verfahrens (c) beschriebenen Komplexe von Metallen der achten Nebengruppe des Periodensystems der Elemente nach Mendelejew in Frage.

Als Wasserstoffdonatoren kommen hierbei primäre und sekundäre, ein- oder mehrwertige Alkohole in Betracht. Vorzugsweise verwendbar sind Methanol, Ethanol, n-Propanol, i-Propanol, n-Butanol, 2-Butanol, Benzylalkohol, Ethylenglykol, 1,3-Propandiol, 1,4-Butandiol und 1,5-Pentandiol. Diese Alkohole können sowohl als Wasserstoffdonatoren als auch als Lösungsmittel dienen.

Weitere Wasserstoffdonatoren, die in der zweiten Stufe des Verfahrens (c) eingesetzt werden können, sind Alkalimetall- und Erdalkalimetall-Salze der Ameisensäure, wie Natriumformiat und Kaliumformiat, und auch die Ameisensäure selbst. - Bei Verwendung eines Salzes der Ameisensäure kann die zweite Stufe des Verfahrens (c) in Form einer Phasen-transfer-Katalyse durchgeführt werden, wobei das Cyclopropylketon der Formel (XI) und der Hydrierkatalysator in einem geeigneten inerten Solvens gelöst sind und das Formiat als wäßrige Lösung in einer zweiten Phase vorliegt. Geeignete Solventien sind deshalb in diesem Zusammenhang solche Lösungsmittel, die einerseits das Cyclopropylketon der Formel (XI) und den Hydrierkatalysator lösen, andererseits aber nicht mit Wasser mischbar sind. Derartige Lösungsmittel sind z.B. Benzol, Toluol, Chlorbenzol, Dichlorbenzol und Methylenchlorid. - Als Phasen-transfer-Katalysatoren kommen alle zu diesem Zweck in der organischen Chemie einsetzbaren Reaktionsbeschleuniger in Betracht. Vorzugsweise verwendbar sind Tetrabutyl-ammonium-bromid und Methyltridecyl-ammonium-chlorid (Aliquat®336).

Die für die zweite Stufe des Verfahrens (c) erforderliche Reaktionszeit ist abhängig von der Reaktionstemperatur, dem Wasserstoffpartialdruck, der Intensität der Durchmischung des Reaktionsgemisches und von der Aktivität und Konzentration des Hydrierkatalysators. Im allgemeinen liegt die erforderliche Reaktionszeit im Bereich von 15 Minuten bis zu mehreren Stunden. - Die Aufarbeitung erfolgt jeweils nach üblichen Methoden.

Die außerdem bei dem Verfahren (b) als Ausgangsstoffe benötigten Verbindungen der Formel (VIII) sind allgemein bekannte Verbindungen der organischen Chemie.

Als Säurebindemittel kommen bei der Durchführung des Verfahrens (b) alle üblichen Säureakzeptoren in Betracht. Vorzugsweise verwendbar sind Alkalimetallcarbonate und Hydrogencarbonate, wie Natriumcarbonat, Kaliumcarbonat oder Natriumhydrogencarbonat, ferner Alkalimetallhydroxide und -Alkoholate, wie Natriumhydroxid, Kaliumhydroxid, Natriummethylat oder Kalium-tert.-butylat, außerdem tertiäre aliphatische oder aromatische Amine, wie Triethylamin, N,N-Dimethyl-cyclohexyl-amin, N,N-Dimethyl-benzylamin und Pyridin, und außerdem cyclische Amine, wie 1,5-Diaza-bicyclo [4.3.0]non-5-en (DBN), 1,8-Diaza-bicyclo-[5.4.0]undec-7-en (DBU) und 1,4-Diaza-bicyclo[2.2.2]octan (DABCO).

Als Verdünnungsmittel kommen bei der Durchführung des Verfahrens (b) alle inerten organischen Solventien in Betracht. Vorzugsweise verwendbar sind aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Dimethyl- und Dibutylether, Glykoldimethylether und Diglykoldimethylether, Tetrahydrofuran und Dioxan, Ketone, wie Aceton, Methylethyl-, Methyl-isopropyl- und Methyl-isobutyl-keton, Ester, wie Essigsäuremethylester und -ethylester, Nitrile, wie z.B. Acetonitril und Propionitril und Pyridin, sowie auch stark polare Lösungsmittel, wie Dimethylformamid und Dimethylsulfoxid.

Die Reaktionstemperaturen können bei dem Verfahren (b) innerhalb eines größeren Bereiches variiert werden. Im allgemeinen arbeitet man bei Temperaturen zwischen 0°C und 150°C, vorzugsweise zwischen 20°C und 130°C.

Bei der Durchführung des Verfahrens (b) setzt man auf 1 Mol an Halogenketon der Formel (VII) im allgemeinen 1 bis 1,5 Mol an Verbindungen der Formel (VIII) sowie 1 bis 2 Mol an Säurebindemittel ein. Die Aufarbeitung erfolgt nach üblichen Methoden. Im allgemeinen geht man so vor, daß man gegebenenfalls nach vorherigem Abfiltrieren von abgeschiedenen Salzen das Reaktionsgemisch einengt, den Rückstand in einem mit Wasser wenig mischbaren organischen Lösungsmittel aufnimmt, die entstehende Lösung wäscht, trocknet und dann einengt.

Das bei dem Verfahren (a) als Reaktionskomponente benötigte Dimethyl-oxo-sulfonium-methylid der Formel (V) ist bekannt (vgl. J. Am. Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, in Gegenwart eines Verdünnungsmittels erzeugt.

Das bei dem Verfahren (a) außerdem als Reaktionskomponente in Betracht kommende Dimethylsulfonium-methylid der Formel (VI) ist ebenfalls bekannt (vgl. Heterocycles 8, 397 (1977)). Es wird bei der obigen Umsetzung gegebenenfalls in frisch hergestelltem Zustand eingesetzt, indem man es in situ z.B. aus Trimethylsulfoniumhalogenid oder Trimethylsulfonium-methylsulfat, in Gegenwart einer starken

Base, wie z.B. Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (a) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei der Durchführung des Verfahrens (a) in einem größeren Bereich variiert werden. Im allgemeinen arbeitet man zwischen 0 °C und 100 °C, vorzugsweise zwischen 10 °C und 60 °C.

Bei der Durchführung des Verfahrens (a) setzt man auf 1 Mol an Cyclopropyl-keton der Formel (IV) im allgemeinen 1 bis 3 Mol an Dimethyloxosulfonium-methylid der Formel (V) bzw. an Dimethylsulfonium-methylid der Formel (VI) ein. Die Isolierung der Oxirane der Formel (II) erfolgt nach üblichen Methoden.

Das bei der Durchführung des erfindungsgemäßen Verfahrens als Reaktionskomponente benötigte 1,2,4-Triazol der Formel (III) ist eine allgemein bekannte Verbindung der organischen Chemie.

Als Säurebindemittel kommen bei der Durchführung des erfindungsgemäßen Verfahrens alle üblichen Säureakzeptoren in Frage. Vorzugsweise verwendbar sind Alkalimetallcarbonate und Hydrogencarbonate, wie Natriumcarbonat, Kaliumcarbonat oder Natriumhydrogencarbonat, ferner Alkalimetallhydroxide und -Alkoholate, wie Natriumhydroxyid, Kaliumhydroxid, Natriummethylat oder Kaliumtert.-butylat, außerdem tertiäre aliphatische oder aromatische Amine, wie Triethylamin, N,N-Dimethyl-cyclohexyl-amin, N,N-Dimethyl-benzylamin und Pyridin, und außerdem cyclische Amine, wie 1,5-Diaza-bicyclo[4.3.0]non-5-en (DBN), 1,8-Diaza-bicyclo[5.4.0]undec-7-en (DBU) und 1,4-Diaza-bicyclo[2.2.2]octan (DABCO).

Als Verdünnungsmittel kommen bei der Durchführung des erfindungsgemäßen Verfahrens alle üblichen inerten organischen Solventien in Betracht. Vorzugsweise verwendbar sind Nitrile, wie insbesondere Acetonitril; aromatische Kohlenwasserstoffe, wie Benzol, Toluol und Dichlorbenzol; Formamide, wie insbesondere Dimethylformamid, sowie Hexamethylphosphorsäuretriamid.

Die Reaktionstemperaturen können bei der Durchführung des erfindungsgemäßen Verfahrens in einem größeren Bereich variiert werden. Im allgemeinen arbeitet man bei Temperaturen zwischen 0 °C und 200 °C, vorzugsweise zwischen 50 und 150 °C.

Bei der Durchführung des erfindungsgemäßen Verfahrens setzt man vorzugsweise auf 1 Mol an Oxiran der Formel (II) 1 bis 4 Mol an 1,2,4-Triazol der Formel (III) und 1 bis 2 Mol an Base ein. Die Isolierung der Endprodukte erfolgt in üblicher Weise.

Die nach dem erfindungsgemäßen Verfahren erhältlichen Stoffe der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol, und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmcdiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;

Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreidekrankheiten, wie Erysiphe graminis, Puccinia recondita, Cochliobolus sativus, Pyrenophora teres, Leptosphaeria nodorum und Gerstenmehltau; ferner von Reiskranheiten, wie Pyricularia oryzae und Pellicularia sasakii; sowie von Venturia-Arten und Gurkenmehltau. Die Stoffe besitzen außerdem eine sehr gute in-vitro-Wirkung.

Die Wirkstoffe können in die üblichen Formulierungen übergeführt werden, die Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streck mitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Träger stoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: Z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmenge kann je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmenge von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Wirkstoffe geht aus den nachfolgenden Beispielen hervor.

## Herstellungsbeispiele

### Beispiel 1

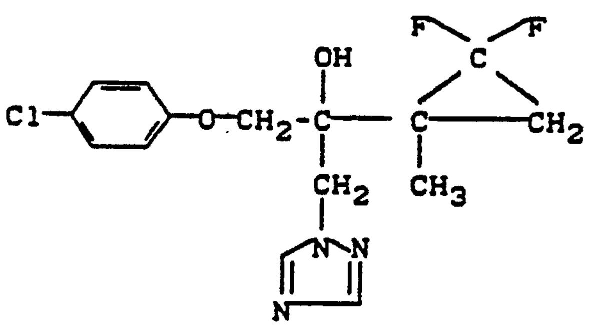

(I-1)

4,2 g (0,015 Mol) 2-(4-Chlorphenoxymethyl)-2-(2,2-difluor-1-methylcyclopropyl)-oxiran, 2,1 g (0,03 Mol) 1,2,4-Triazol und 4,2 g (0,03 Mol) Kaliumcarbonat werden in 30 ml Dimethylformamid 16 Stunden bei 90 °C gerührt. Nach dem Entfernen des Lösungsmittels an der Ölpumpe wird der Rückstand mit Wasser/Methylenchlorid verrührt, die organische Phase abgetrennt und eingeengt. Der Rückstand wird mittels Säulenchromatographie (Chloroform/Essigester = 4:1) an Kieselgel gereinigt.

Man erhält 2,3 g (44,6 % der Theorie) 1-(4-Chlorphenoxy)-2-(2,2-difluor-1-methylcyclopropyl)-2-hydroxy-3-(1,2,4-triazol-1-yl)-propan vom Schmelzpunkt 110 °C.

### Herstellung von Ausgangssubstanzen

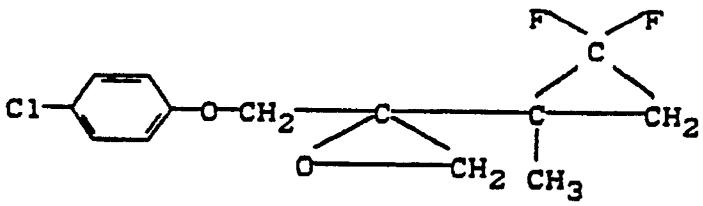

(II-1)

Zur Lösung von 30 ml (0,04 Mol) einer 1,36 molaren Lösung von Trimethylsulfonium-methylsulfat in Acetonitril fügt man 2,4 g (0,044 Mol) Natriummethylat hinzu und rührt 30 Minuten bei Raumtemperatur. Dann setzt man 5,8 g (0,022 Mol) 1-(4-Chlorphenoxyacetyl)-1-methyl-2,2-difluorcyclopropan zu und läßt 16 Stunden bei 20° C rühren. Man gießt das Reaktionsgemisch in 200 ml Wasser, extrahiert mit Methylenchlorid, wäscht die organische Phase mit Wasser und engt die organische Phase ein. Man erhält 4,3 g (GC-Gehalt von 73 %; 71,6 % der Theorie) 2-(4-Chlorphenoxymethyl)-2-(2,2-difluor-1-methylcyclopropyl)-oxiran als zähes Harz, das direkt weiter umgesetzt wird.

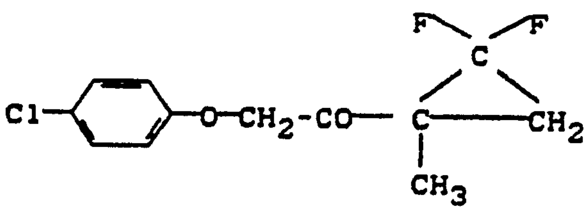

(IV-1)

6 g (0,028 Mol) 1-Bromacetyl-1-methyl-2,2-difluorcyclopropan werden in 30 ml Aceton unter Zusatz von 4,4 g (0,032 Mol) Kaliumcarbonat und 4,1 g (0,032 Mol) 4-Chlorphenol 16 Stunden unter Rühren unter Rückfluß erhitzt. Man verdünnt mit Wasser, extrahiert das Produkt mit Methylenchlorid, wäscht die organische Phase einmal mit verdünnter Natronlauge, dann mit Wasser und engt unter vermindertem Druck ein.

Man erhält 6 g 1-(4-Chlorphenoxyacetyl)-1-methyl-2,2-difluorcyclopropan als Rohprodukt mit einem Gehalt (GC) von 66 % (54,4 % der Theorie).

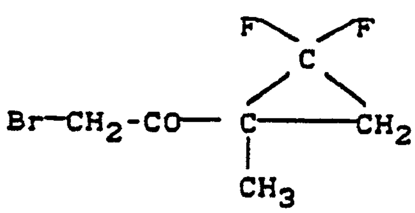

(VII-1)

Zur Lösung von 12,5 g (0,09 Mol) 1-Acetyl-1-methyl-2,2-difluor-cyclopropan in 50 ml Methanol tropft man unter Rühren bei 20° C eine Lösung von 4,7 ml (0,09 Mol) Brom in 30 ml Methylenchlorid. Nach vollständiger Entfärbung gießt man das Reaktionsgemisch in 200 ml Wasser und extrahiert mit Methylenchlorid. Nach dem Einengen unter vermindertem Druck erhält man 20 g (GC-Gehalt 47 %; 47 % der Theorie) rohes 1-Bromacetyl-1-methyl-2,2-difluor-cyclopropan, das ohne weitere Reinigung direkt umgesetzt wird.

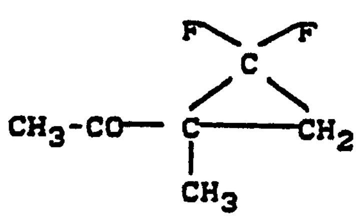

(X-1)

Zu 34 g (0,25 Mol) 2,2-Difluor-1-methylcyclopropancarbonsäure in 250 ml trockenem Diethylether tropft man unter Rühren bei -78° C unter Stickstoff 333 ml einer 1,5-molaren Methyllithiumlösung (0,5 Mol). Man

rührt noch eine Stunde bei -78° C nach, erwärmt dann auf 0° C und gießt die Reaktionslösung auf 500 g Eis und 50 ml konzentrierter Salzsäure. Die organische Phase wird abgetrennt und die wäßrige Phase mit Ether extrahiert. Die vereinigten organischen Phasen werden über Natriumsulfat getrocknet, das Lösungsmittel abdestilliert und das Produkt wird unter leicht vermindertem Druck destilliert.

Man erhält 21 g (63 % der Theorie) 2,2-Difluor-1-methylcyclopropylmethylketon vom Siedepunkt 58-60° C/60 mbar.

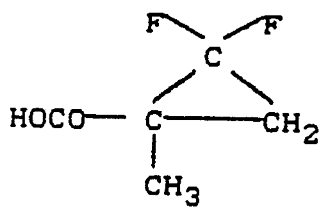

(XIII-1)

840 g (7,12 Mol) 2,2-Difluor-1-methyl-1-vinyl-cyclopropan in 10 l Wasser werden portionsweise mit 2,3 kg (14,47 Mol) Kaliumpermanganat versetzt. Man läßt 36 Stunden bei Raumtemperatur rühren, filtriert von Braunstein ab und wäscht gut mit Wasser nach. Das Filtrat wird mit konzentrierter Salzsäure sauer gestellt und mit Dichlormethan extrahiert. Nach dem Trocknen der organischen Phase wird das Lösungsmittel unter vermindertem Druck entfernt und der Rückstand destilliert.

Man erhält so 750 g (77 % der Theorie) 2,2-Difluor-1-methylcyclopropancarbonsäure (77 % der Theorie) vom Schmelzpunkt 59-61° C.

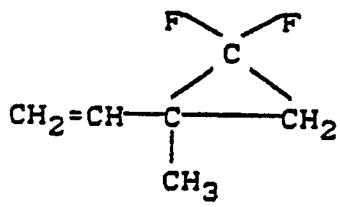

(XII-1)

Die Addition von Difluorcarben an Isopren unter Bildung von 2,2-Difluor-1-methyl-1-vinyl-cyclopropan ist bereits bekannt und beschrieben von M.Kamel, W.Kimpenhaus, J.Buddrus, Chem. Ber. 109, 2351 (1976).

Beispiel 2

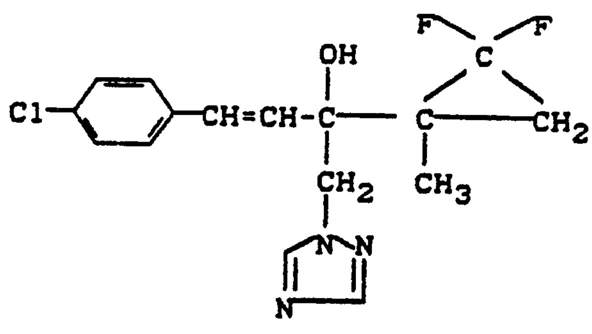

(I-2)

Zur Lösung von 3,3 g (0,048 Mol) 1,2,4-Triazol in 30 ml Dimethylformamid fügt man portionsweise 1,4 g (0,048 Mol) 80 %iges Natriumhydrid und rührt 30 Minuten bei 20-30° C. Anschließend setzt man 6,6 g (0,024 Mol) 2-(2,2-Difluor-1-methyl-cyclopropyl)-2-[2-(4-chlorphenyl)-ethen-1-yl]-oxiran zu und läßt 16 Stunden bei 90° C rühren. Man gießt das Reaktionsgemisch dann auf Wasser, extrahiert mit Methylenchlorid, engt die organische Phase unter vermindertem Druck ein und reinigt den Rückstand durch Chromatographie an Kieselgel (Chloroform/Essigester = 4:1).

Man erhält 2,4 g (29,6 % der Theorie) 1-(4-Chlorphenyl)-3-(2,2-difluor-1-methylcyclopropyl)-3-hydroxy-4-(1,2,4-triazol-1-yl)-but-1-en vom Schmelzpunkt 131° C.

Herstellung von Ausgangssubstanzen

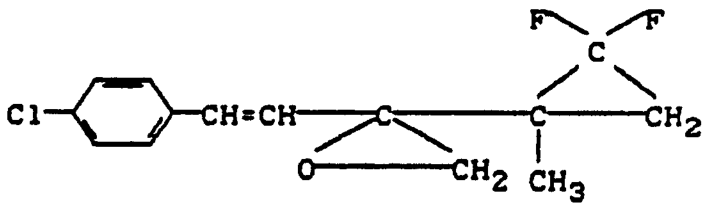

(II-2)

41 ml (0,06 Mol) einer 1,46-molaren Lösung von Trimethylsulfoniummethylsulfat in Acetonitril werden mit 3,6 g (0,066 Mol) Natriummethylat versetzt und 30 Minuten bei Raumtemperatur gerührt. Anschließend fügt man eine Lösung von 10 g (0,039 Mol) (2,2-Difluor-1-methyl-cyclopropyl)-[(2-(4-chlorphenyl)-ethen-1-yl]-keton in 100 ml Acetonitril hinzu und rührt 16 Stunden bei Raumtemperatur. Man gießt dann das Reaktionsgemisch in Wasser, extrahiert mit Methylenchlorid, wäscht die organische Phase zweimal mit Wasser und engt die organische Phase unter vermindertem Druck ein.

Man erhält 6,8 g (GC-Gehalt 38 %; 24 % der Theorie) 2-(2,2-Difluor-1-methylcyclopropyl-2-[2-(4-chlorphenyl)-ethen-1-yl]-oxiran, das ohne weitere Reinigung direkt umgesetzt wird.

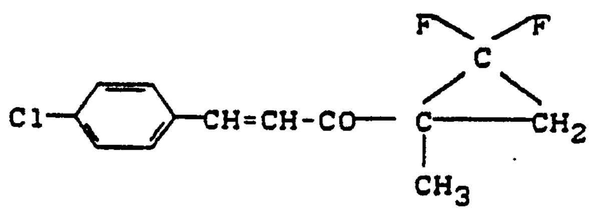

(IV-2)

15 g (0,11 Mol) 1-Acetyl-2,2-difluor-1-methylcyclopropan in 20 ml 10%-iger methanolischer Natronlauge werden unter Rühren mit einer Lösung von 15,5 g (0,11 Mol) p-Chlorbenzaldehyd in 20 ml Methanol bei 20 °C versetzt. Nach 15 Minuten fügt man weitere 40 ml 10 %-ige methanolische Natronlauge zu und rührt weitere 16 Stunden bei Raumtemperatur. Man gießt das Reaktionsgemisch in Wasser, extrahiert mit Methylenchlorid, wäscht die organische Phase mit Wasser und engt unter vermindertem Druck ein.

Man erhält 25 g (88 % der Theorie) rohes (2,2-Difluor-1-methylcyclopropyl)-[2-(4-chlorphenyl)-ethen-1-yl]-keton als Öl, das direkt weiter umgesetzt wird.

Entsprechend den Beispielen 1 und 2 sowie gemäß den angegebenen Verfahrensbedingungen werden die in der folgenden Tabelle 2 aufgeführten Stoffe erhalten:

Tabelle 2

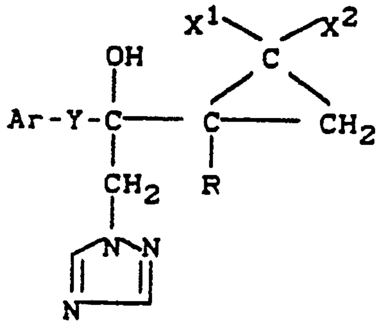 (I)

| Verbindungs-Nr. | Ar | Y | R | $X^1$ | $X^2$ | physikal. Konstante Fp(°C) |
|---|---|---|---|---|---|---|
| I-3 | 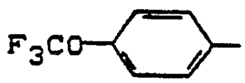 | $-OCH_2-$ | $CH_3$ | F | F | Harz |
| I-4 | 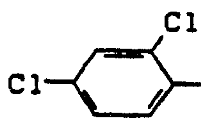 | $-OCH_2-$ | $CH_3$ | F | F | Harz |
| I-5 | 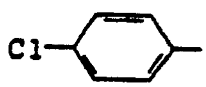 | $-CH_2CH_2-$ | $CH_3$ | F | F | 112 |
| I-6 | 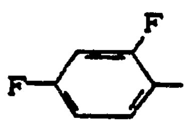 | $-OCH_2-$ | $CH_3$ | F | F | Harz |
| I-7 | 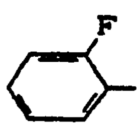 | $-OCH_2-$ | $CH_3$ | F | F | Harz |
| I-8 | 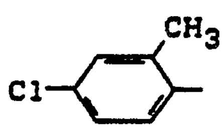 | $-OCH_2-$ | $CH_3$ | F | F | Harz |

| Verbindungs-Nr. | Ar | Y | R' | $X^1$ | $X^2$ | physikal. Konstante Fp(°C) |
|---|---|---|---|---|---|---|
| I-9 | 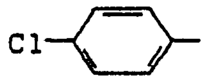 | $-OCH_2-$ | $CH_3$ | Cl | Cl | Harz |
| I-10 | 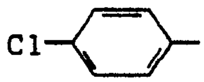 | $-CH_2CH_2-$ | $CH_3$ | Cl | Cl | 109-112 |
| I-11 | 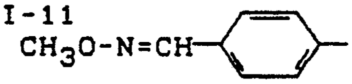 | $-OCH_2-$ | $CH_3$ | F | F | Harz |
| I-12 | 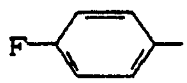 | $-OCH_2-$ | $CH_3$ | F | F | Harz |
| I-13 | 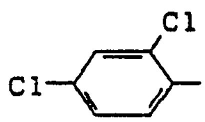 | -CH=CH- | $CH_3$ | F | F | Harz |

In den folgenden Verwendungsbeispielen wurden die Verbindungen der nachstehend angegebenen Formeln als Vergleichssubstanzen eingesetzt:

(A) =

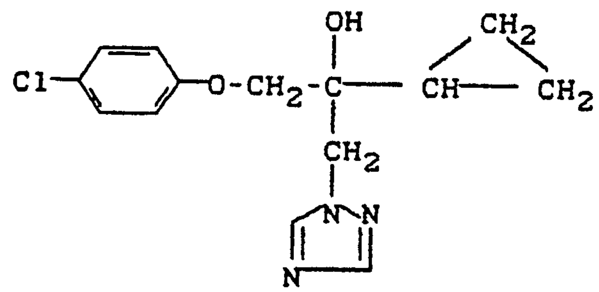

(Bekannt aus EP-OS 0 040 345)

(B) =

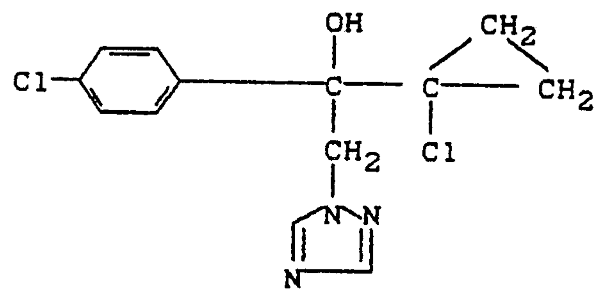

(Bekannt aus EP-OS 0 180 136)

(C) =

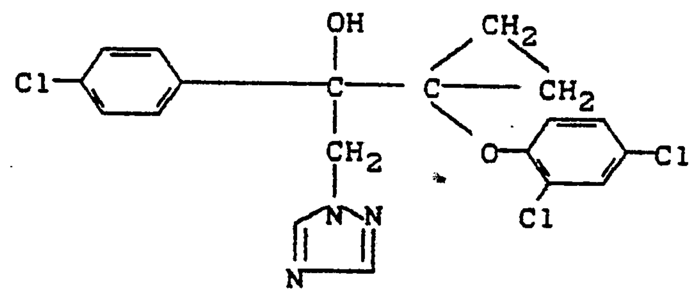

(Bekannt aus EP-OS 0 180 136)

Beispiel A

Erysiphe-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe f.sp.tritici bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen (I-1), (I-2), (I-3) und (I-4) eine wesentlich

bessere Wirksamkeit als die Vergleichssubstanzen (B) und (C).

Beispiel B

Puccinia-Test (Weizen) / protektiv

Lösungsmittel: 100 Gewichtsteile Dimethylformamid
Emulgator: 0,025 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita mit in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20° C und 100 % rel. Luftfeuchtigkeit in einer Inkubationskabine.

Die Pflanzen werden in einem Gewächshaus bei einer Temperatur von ca. 20 ° C und einer relativen Luftfeuchtigkeit von ca. 80 % aufgestellt, um die Entwicklung von Rostpusteln zu begünstigen.

10 Tage nach derInokulation erfolgt die Auswertung.

In diesem Test zeigen die erfindungsgemäßen Verbindungen (I-1), (I-2), (I-3), (I-4) und (I-5) eine wesentlich bessere Wirksamkeit als die Vergleichssubstanz (A).

Beispiel C

Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20° C und 100 % relativer Luftfeuchtigkeit in einer Inkubationskabine.

Die Pflanzen werden dann im Gewächshaus bei 20 ° C und einer relativen Luftfeuchtigkeit von ca. 70 % aufgestellt.

12 Tage nach der Inokulation erfolgt die Auswertung.

In diesem Test zeigen die erfindungsgemäßen Verbindungen (I-1), (I-2) und (I-5) eine wesentlich bessere Wirksamkeit als die Vergleichssubstanz (C).

## Ansprüche

1. 2,2-Dihalogencyclopropyl-hydroxyethyltriazole der Formel

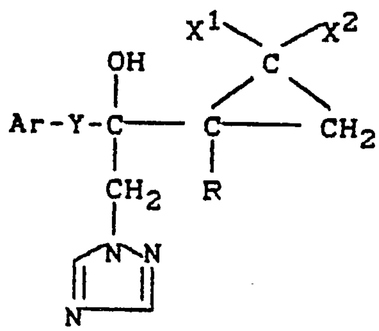

(I)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Halogen steht und
Y für die Gruppierungen $-OCH_2-$, $-SCH_2-$, $-CH_2CH_2-$oder $-CH=CH-$ steht, wobei das Heteroatom mit dem Aryl-Rest verbunden ist, wenn Y für $-OCH_2-$oder $-SCH_2-$steht,
sowie deren Säureadditions-Salze und Metallsalz-Komplexe.

2. Verfahren zur Herstellung von 2,2-Dihalogencyclopropyl-hydroxy-ethyltriazolen der Formel

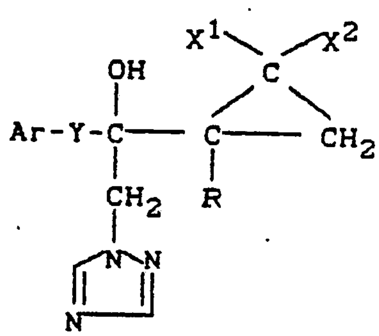

(I)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Halogen steht und
Y für die Gruppierungen $-OCH_2-$, $-SCH_2-$, $-CH_2CH_2-$oder $-CH=CH-$ steht, wobei das Heteroatom mit dem Aryl-Rest verbunden ist, wenn Y für $-OCH_2-$oder $-SCH_2-$steht,
sowie von deren Säureadditions-Salzen und Metallsalz-Komplexen, dadurch gekennzeichnet, daß man Oxirane der Formel

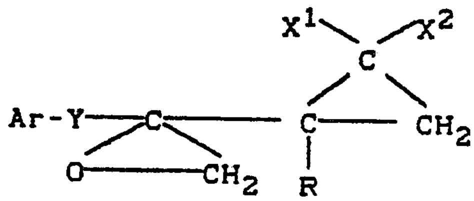

(II)

in welcher
Ar, R, $X^1$, $X^2$ und Y die oben angegebenen Bedeutungen haben,
mit 1,2,4-Triazol der Formel

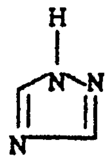

(III)

in Gegenwart eines Säurebindemittels und in Gegenwart eines Verdünnungsmittels umsetzt und gegebenenfalls anschließend an die so erhältlichen Verbindungen der Formel (I) eine Säure oder ein Metallsalz addiert.

3. Fungizide Mittel, gekennzeichnet, durch einen Gehalt an mindestens einem 2,2-Dichlorhalogencyclopropyl-hydroxyethyltriazol der Formel (I) gemäß Anspruch 1 bzw. einem Säureadditions-Salz oder Metallsalz-Komplex eines 2,2-Dihalogencyclopropylhydroxy-ethyltriazols der Formel (I).

4. Verwendung von 2,2-Dihalogencyclopropyl-hydroxy-ethyltriazolen der Formel (I) gemäß Anspruch 1 bzw. von deren Säureadditions-Salzen oder Metallsalz-Komplexen zur Bekämpfung von Pilzen.

5. Oxirane der Formel

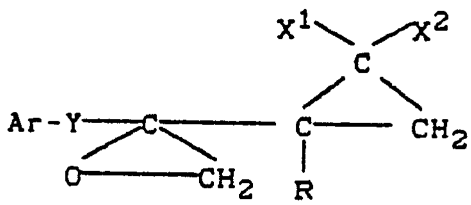

(II)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Halogen steht und
Y für die Gruppierungen $-OCH_2-$, $-SCH_2-$, $-CH_2CH_2-$oder $-CH=CH-$ steht, wobei das Heteroatom mit dem Aryl-Rest verbunden ist, wenn Y für $-OCH_2-$oder $-SCH_2-$steht.

6. Verfahren zur Herstellung von Oxiranen der Formel

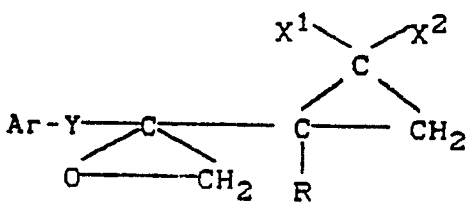

(II)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Halogen steht und
Y für die Gruppierungen $-OCH_2-$, $-SCH_2-$, $-CH_2CH_2-$oder $-CH=CH-$ steht, wobei das Heteroatom mit dem Aryl-Rest verbunden ist, wenn Y für $-OCH_2-$oder $-SCH_2-$steht,
dadurch gekennzeichnet, daß man
a) Cyclopropylketone der Formel

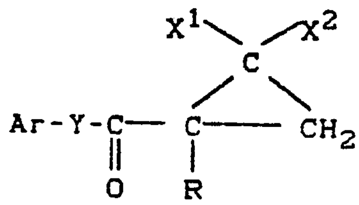

(IV)

in welcher
Ar, R, $X^1$, $X^2$ und Y die oben angegebene Bedeutung haben,
entweder
α) mit Dimethyloxosulfonium-methylid der Formel

$$(CH_3)_2\overset{\delta^{\oplus}}{S}O\overset{\delta^{\ominus}}{C}H_2 \quad . \qquad (V)$$

oder
β) mit Dimethylsulfonium-methylid der Formel

$$(CH_3)_2\overset{\delta^{\oplus}}{S}\ \overset{\delta^{\ominus}}{C}H_2 \qquad (VI)$$

in Gegenwart eines Verdünnungsmittels umsetzt.

7. Cyclopropylketone der Formel

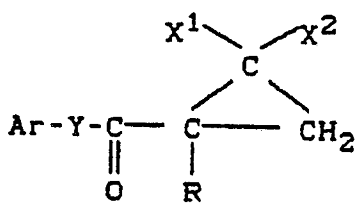

(IV)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Halogen steht und
Y für die Gruppierungen $-OCH_2-$, $-SCH_2-$, $-CH_2CH_2-$oder $-CH=CH-$ steht, wobei das Heteroatom mit dem Aryl-Rest verbunden ist, wenn Y für $-OCH_2-$oder $-SCH_2-$steht.

8. Verfahren zur Herstellung von Cyclopropylketonen der Formel

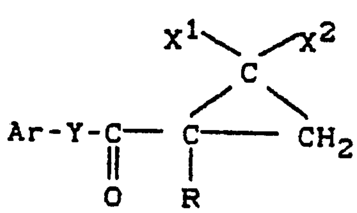

(IV)

Ar für gegebenenfalls substituiertes Aryl steht,
R für Alkyl, gegebenenfalls substituiertes Aryl oder gegebenenfalls substituiertes Aralkyl steht,

$X^1$ für Halogen steht,
$X^2$ für Halogen steht und
Y für die Gruppierungen $-OCH_2-$, $-SCH_2-$, $-CH_2CH_2-$oder $-CH=CH-$ steht, wobei das Heteroatom mit dem Aryl-Rest verbunden ist, wenn Y für $-OCH_2-$oder $-SCH_2-$steht,
dadurch gekennzeichnet, daß man
b) Halogenketone der Formel

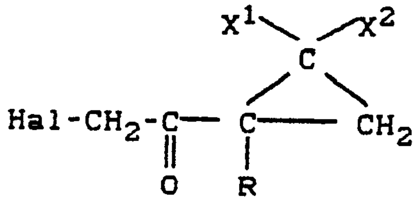

(VII)

in welcher
Hal für Chlor oder Brom steht und
R, $X^1$ und $X^2$ die oben angegebenen Bedeutungen haben,
mit Verbindungen der Formel
Ar - Z - H (VIII)
in welcher
Ar die oben angegebene Bedeutung hat und
Z für Sauerstoff oder Schwefel steht,
in Gegenwart eines Säurebindemittels und gegebenenfalls in Gegenwart eines Verdünnungsmittels umsetzt; oder
c) Aldehyde der Formel
Ar - CHO (IX)
in welcher
Ar die oben angegebene Bedeutung hat,
mit Methyl-cyclopropyl-ketonen der Formel

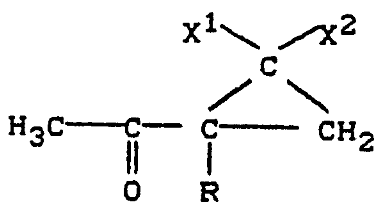

(X)

in welcher
R, $X^1$ und $X^2$ die oben angegebenen Bedeutungen haben,
in Gegenwart eines Katalysators und in Gegenwart eines Verdünnungsmittels umsetzt und gegebenenfalls die dabei entstehenden Cyclopropylketone der Formel

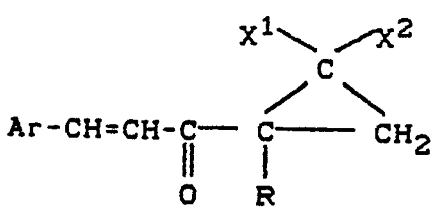

(XI)

in welcher
Ar, R, $X^1$ und $X^2$ die oben angegebenen Bedeutungen haben,
in Gegenwart eines Katalysators und in Gegenwart eines Verdünnungsmittels hydriert.

9. Halogenketone der Formel

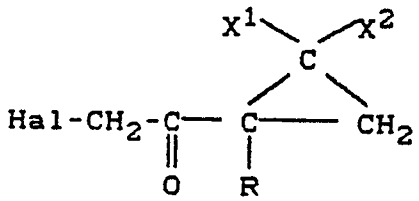

(VII)

in welcher
Hal für Chlor oder Brom steht,
R für Alkyl, gegebenenfalls substituiertes Aryl oder gegebenenfalls substituiertes Aralkyl steht,
$X^1$ für Halogen steht und
$X^2$ für Halogen steht.

10. Verfahren zur Herstellung von Halogenketonen der Formel

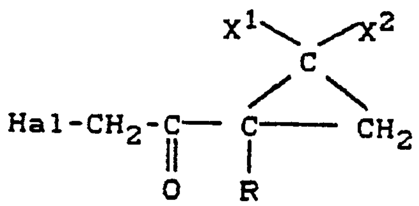

(VII)

in welcher
Hal für Chlor oder Brom steht,
R für Alkyl, gegebenenfalls substituiertes Aryl oder gegebenenfalls substituiertes Aralkyl steht,
$X^1$ für Halogen steht und
$X^2$ güt Halogen steht,
dadurch gekennzeichnet, daß man
d) Methyl-cyclopropyl-ketone der Formel

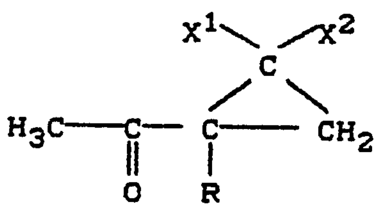

(X)

in welcher
R, $X^1$ und $X^2$ die oben angegebenen Bedeutungen haben,
mit Chlorierungsmitteln oder Bromierungsmitteln in Gegenwart eines Verdünnungsmittels umsetzt.